# EUROPEAN PATENT APPLICATION

(11) **EP 4 685 767 A1**
(43) Date of publication of application: **28.01.2026**
(21) Application number: 24190501.7
(22) Date of filing: 24.07.2024
(51) Int. Cl.: G08B 21/04, G08B 29/18, G08B 31/00, A61B 5/11

(54) **PRIVACY-PRESERVING SMART HOME SOLUTIONS TO ASSIST OLDER ADULTS IN HIGH-RISK SITUATIONS THROUGH DIGITAL PREDICTIONS AND INTERVENTIONS**

(71) Applicant: Serai AG, 8008 Zurich (CH)
(72) Inventor: The designation of the inventor has not yet been filed
(74) Representative: Bakirci, Utkan Bahri

(57) **Abstract**

The present invention relates to a smart living system specifically designed for older adults and senior care, utilizing artificial intelligence to optimize its performance. The system provides a secure living environment with a focus on the health and wellness of its occupants. The invention employs, a deep learning-based solution to address the primary factors affecting the physical and mental well-being of older adults. In particular, the present invention relates to AI-enabled living spaces that use novel algorithms to collect, process, analyze and recommend sensor data. The system comprises precision sensors, actuators, smart devices, mobile applications, AI algorithms, and other components that synergistically work together to generate insights, thereby optimizing the user's living environment, improving the system's efficiency and reducing its environmental impact. The system is highly adaptable and can be customized to meet the unique needs of each older adult. Additionally, the invention relates to novel housing systems specifically designed for AI-enabled living spaces, ensuring broad applicability across various types of senior care facilities.

## Description

The present invention relates to a smart living system specifically designed for older adults and senior care, utilizing artificial intelligence to optimize its performance. The system provides a secure living environment with a focus on the health and wellness of its occupants. The invention employs, a deep learning-based solution to address the primary factors affecting the physical and mental well-being of older adults. In particular, the present invention relates to Al-enabled living spaces that use novel algorithms to collect, process, analyze and recommend sensor data. The system comprises precision sensors, actuators, smart devices, mobile applications, AI algorithms, and other components that synergistically work together to generate insights, thereby optimizing the user's living environment, improving the system's efficiency and reducing its environmental impact. The system is highly adaptable and can be customized to meet the unique needs of each older adult. The present invention is also applicable to nursing homes, hospitals, senior homes, and conventional apartment flats, provided that the building is suitable for technological integration. Additionally, the invention relates to novel housing systems specifically designed for Al-enabled living spaces, ensuring broad applicability across various types of senior care facilities.

In recent years, the world has experienced a rapid and unprecedented pace of change, driven by a range of factors including the COVID-19 pandemic, rapid urbanization, the effects of climate change, the increase in the elderly population and the rise of new technologies. Especially the pandemic has had a profound impact on every aspect of life, from healthcare and the economy to social and cultural norms, forcing us to rethink our ways of living and working.

Furthermore, the gradual increase in the elderly population has posed a common challenge with far-reaching implications for healthcare systems, social and economic structures, and the overall well-being of older adults. As individuals age, they become more susceptible to chronic health conditions, leading to a higher demand for healthcare services. This trend has placed a significant strain on healthcare systems, resulting in an increased demand for healthcare professionals. Additionally, the rising prevalence of chronic health conditions has resulted in escalating healthcare costs, as more individuals require medical treatments and services, thereby exacerbating the burden on public healthcare systems. Furthermore, the aging population has also led to an increased demand for assisted living facilities and long-term care facilities, leading to a shortage of available facilities and increased costs for individuals and families. Addressing these challenges requires innovative solutions and a comprehensive approach that caters to the evolving needs of the aging population. The demand for healthcare and assisted living services is high, and older adults require assistance with daily tasks, monitoring their health, and receiving support during emergencies. Therefore, it is critical to develop innovative solutions that align with the needs of the aging population to ensure that they receive the necessary support and care.

Thus, aging population and their living environment is a serious problem that should be addressed. For instance, Switzerland exhibits a demographic composition as of 2022, where 19.3% of the country's population is composed of individuals aged 65 years and older. Aligned with international standards, the Swiss government officially defines people aged 65 years and over as having entered the elderly phase, and projections indicate that the demographic proportion within this age bracket is expected to escalate to 23.04% by the year 2030, posing significant challenges to the healthcare system. Consequently, one can infer from this data that Switzerland is experiencing demographic aging, like other members of the Organization for Economic Cooperation and Development (OECD). Primarily driven by low fertility rates, increased life expectancy, and migration. In the early 20th century, the average Swiss woman had 3.7 children, but this number has now dropped to 1.5, leading to fewer young people in society. Concurrently, the average life expectancy has doubled since 1878, contributing to an increase in the elderly population. A significant demographic trend in Switzerland relates to the high occurrence of elderly people living alone. Observations indicate that a notable 35.2% of elderly individuals in Switzerland choose to reside in solitary living arrangements, marking one of the higher percentages among European countries. This living arrangements of elderly individuals are a result of personal preferences, financial resources, and the impact of social, economic, and health factors that arise as they age. For instance, countries like Sweden and Denmark exhibit even higher proportions of elderly people residing alone, often surpassing 40%. These nations are recognized for their robust social welfare systems and a cultural emphasis on individual self-reliance. The gender-related factor is unmistakably apparent when considering the solitary living arrangements of older individuals in Switzerland. Switzerland exhibits one of the most pronounced gender gaps in Europe, with a notable 28-percentage-point difference, where a higher proportion of older women live alone compared to their male counterparts. This discrepancy can be ascribed to several factors, including women's longer life expectancy, higher rates of widowhood among women, and a greater tendency for men to remarry following widowhood or divorce. In conclusion, the solitary living arrangements of older individuals in Switzerland are significantly influenced by cultural values emphasizing independence, and personal circumstances such as widowhood, divorce, and family dynamics.

The Swiss Federal Statistical Office (FSO) regularly disseminates statistical data regarding the living arrangements of the elderly population in Switzerland, revealing that 96% of individuals aged 65 and older reside in their own homes. Among the subset of individuals aged 65 to 79 who live in their own homes, it is noteworthy that 25.2% grapple with multiple chronic diseases, while an additional 15% to 25% contend with at least one mental health disorder. According to FSO statistics, in 2017, a significant portion of individuals aged 65 and older in Switzerland sought assistance for health-related reasons, with 15% relying on informal care and 8% utilizing home care services. In contrast, a substantial 31% of older individuals actively provided support to their relatives or acquaintances. It is noteworthy that over 70% of the 370,000 individuals receiving professional home care services were aged 65 or older, and the number of care hours received tended to increase with advancing age. It is reported that in Switzerland, 81% of severe injuries occur because of falls within home environments. The Swiss Council for Accident Prevention (BFU) emphasizes that the risk of injury due to falls is significantly higher among individuals aged 85 and older. The statistics regarding falls among individuals aged 65 and above are indeed alarming. Each year, approximately 88,000 falls occur within this age group, resulting in 1520 fall-related deaths recorded annually between 2011 and 2015, painting a distressing picture of the scale of this issue. What's more, the associated healthcare costs linked to these falls are substantial, consuming up to 1.5% of the country's total healthcare expenditure, amounting to an estimated cost of around 1.2 billion CHF. This financial burden underscores the critical importance of addressing fall prevention measures and investing in the well-being of this aging population. Furthermore, people aged over 80 face an even higher risk of falls, with a 30% prevalence, emphasizing the urgency of targeted interventions to protect their health and safety.

In Switzerland, about 48% of the population identifies mental health as a significant health issue. Socioeconomic circumstances and personal choices, which often lead seniors to reside by themselves, are key influences on the health issues they face, including their mental health. According to the World Health Organization (WHO), experiencing loneliness and social disconnection substantially heightens the risk of developing mental health problems in the elderly stage of life. A study has been conducted in various developed countries to find out the percentage of older people who will be diagnosed by a doctor with depression, anxiety or another mental health problem by 2021. The United States has the highest rate at around 20%, suggesting that a significant proportion of the older population will be diagnosed with a mental health problem. In Switzerland and Norway, this occurs in 7% of the older population, while the Netherlands has the lowest rate at 6% and Germany at 5%. This is a sociodemographic phenomenon that can affect the mental problems experienced by citizens depending on the country they live in. A study was conducted to investigate the impact of social relationships on the mental health of older people in Switzerland. The study unveiled that social distancing measures enforced during the COVID-19 pandemic had a profound effect on the mental well-being of older individuals, leading to adverse emotional responses and an increased sense of isolation. However, the research also discovered that effective communication during this period of isolation played a crucial role in mitigating the adverse effects of stress. These findings underscore the link between loneliness and social isolation, highlighting the importance of fostering communication to support the elderly population in Switzerland.

The results of these studies indicate that the challenges experienced by the elderly population in Switzerland have been well recognized, with solutions being actively pursued through dedicated research, social responsibility initiatives, and technological advancements. In the context of the present invention, the aim is to provide a solution tailored to address the two most significant issues affecting the mental and physical health of elderly individuals in Switzerland.

Against this backdrop of rapid change and uncertainty and the problems associated with the increasing elderly population, all traditional ways of living on the earth became open to discussion, which makes it inevitable to question the most relevant aspect in relation to this; i.e. conventional settlements inside old buildings, in big cities all around the world. Most of the time these old buildings / apartments are not designed for technological integration for providing smart solutions to people living in these houses. Consequently, the necessity for intelligent living spaces for the changing needs and demands of modern cities and their inhabitants has rendered adaptable solutions indispensable for the transition of living spaces found also inside old buildings. Therefore, there is definitely a need in the state of the art, for adapting the existing living spaces irrespective of them being inside an old building, by integrating smart solutions with a design for older adults living alone. Most of the time it is very difficult to transform the old buildings in order to make them suitable for technological integration; which forces an adaptable and optimized design for older adults.

In the state of the art there has been several attempts to address at least parts of the problems to some extent:
FR2829862A1 disloses, a fall detector system that detects the presence of vital vibrations from the person, and whether a first axis on the person is submitted to a rapid movement. If the axis is submitted to a rapid movement, a time window is opened and the angle to the horizontal determined. If the angle is less than a set level, then a fall is detected.

EP365801 0A1 discloses a method wherein a mobile device obtains a signal indicating an acceleration measured by a sensor over a time period. The mobile device determines an impact experienced by the user based on the signal. The mobile device also determines, based on the signal, one or more first motion characteristics of the user during a time prior to the impact, and one or more second motion characteristics of the user during a time after the impact. The mobile device determines that the user has fallen based on the impact, the one or more first motion characteristics of the user, and the one or more second motion characteristics of the user, and in response, generates a notification indicating that the user has fallen.

CN109035701A discloses an automatic alarm method for safety monitoring of the elderly in the home-based elderly care.

Moreover, there are other problems in residential spaces of today's world. Consumers demand data driven suggestions and decision making in everyday living, including health services and predictive assistance for the elderly. There is no prior art solution dealing with such problems which amount to individuals living disconnected from others.

There is therefore still a need on the market for innovative, adaptable designs and combinations of existing solutions as well as incorporating new technologies to living spaces, which are suitable for the improvement of human life quality of elderly people by providing an alternative way of settlement, without having the disadvantages of the systems of the prior art and without being bound to the limitations of conventional housing systems.

As explained herein before, elderly people make up large segments of the population in Switzerland and many other European countries. Many of them live alone in their homes and have health problems that requires monitoring. Therefore, the main objective with the present invention is to offer an Al-based solution for mental and physical health monitoring of elderly people. The solution for this aim will combine multi-channel data and build a recommendation system for the user.

Current elderly care solutions often lack personalization and fail to fully utilize technology. The present invention aims in development of a system that not only utilizes cutting-edge technology for safety and health monitoring but also tailors its functionalities to the individual needs and preferences of its users. Through continuous learning and adaptation, the solution of the present invention aims to offer a more personalized and effective care experience. Additionally, many existing care models are reactive, addressing issues only after they occur. However, elderly people require a proactive care approach, where potential health and safety risks are identified and addressed before they result in harm. This forward-thinking strategy relies on predictive analytics and behavior monitoring to anticipate and prevent incidents, thereby enhancing the overall effectiveness of elderly care. Moreover, current elderly care practices often lack emphasis on social connectivity and community building. But the significant impact of social relationships on mental health is mostly acknowledged and there is a need to bridge this gap by facilitating easier communication between the elderly, their families, and caregivers. By fostering a stronger sense of community, the solution of the present invention aims to alleviate feelings of loneliness and create a more interconnected and supportive care environment.

Thus, the object of the present invention is to design, build and deploy an adaptable system for creating an autonomous living space and to improve the human life quality of elderly individuals. The objective of these intelligent living spaces is to deliver an entirely personalized lifestyle experience by deriving insights from the user's daily routines. A distinctive aspect of the system is its ability to amalgamate data from various sources, including internal sensors, personal smart devices, and external data, to achieve this aim. This feature enables the system to enhance the user's comfort, minimize environmental impact, ensure their safety, and monitor their health, even when the user is away from their living space.

Another object of the present invention is to make solitary living safer and enhancing quality of life for elderly individuals. The world is aging, more and more elderly people are living alone in the entire world and need help to maintain quality of life.

This object and others are achieved by the present invention which relates to an intelligent and a sustainable home system leveraging machine learning and loT to design, build and deploy a smart living space which use predictive AI technology for older adults. The system of the present invention is suitable and adaptable for all living spaces such as the ones in apartments or in smaller buildings. Thus, the present invention pertains to the provision of a novel life experience through the integration of the intelligent system into living spaces. This is achieved through the use of data-driven decision-making and machine-learning techniques, which are intended to support the aforementioned experience.

Responding to the urgent need for innovation in the senior care sector, the present invention proposes a novel video-based monitoring system that turns ordinary homes to smart homes with cost-effective passive sensor installations. Video-based active and assisted living (AAL) technologies offer unique opportunities as they deliver rich information and have vast potential out of the research laboratories for a reliable and personalized solution for senior care if the privacy issues are properly addressed. As fall-related injuries account for more than 80% of severe injuries in older adults, in the scope of the present invention the focus is on rapid fall prevention, detection, and handling by monitoring, analyzing, and interpreting daily activities and potential safety risks at home, while addressing the challenges identified in the current state of the art (e.g., occlusion, low-light conditions). Methodologically, the latest research in (generative) Al, computer vision, and privacy-preserving technology design is leveraged.

The focus of the present invention is twofold: (1) enable effective, scalable, accessible and privacy-preserving technology solutions for fall detection and prevention utilizing off-the-shelf sensors and ubiquitous wireless infrastructure, and (2) improve the user experience pertinent to privacy and trust, the two major hindrances of the technology acceptance, adoption, and use at home.

By integrating sophisticated technology research with human-centered design methodologies, a personalized approach that respects the privacy and autonomy of older adults while providing caregivers with peace of mind is uniquely created. The innovative solution of the present invention represents a significant step forward in redefining senior care standards in Switzerland and the rest of the world, contributing to a safer, more inclusive, and healthier society.

While there are some existing solutions that are in the field of housing systems, none of them provide the same combination of features and benefits as the present invention. The present invention is a smart living space designed specifically for older adults, which is preferably a living system in order to provide an Al-enabled living space without being bound and limited by the conventional housing systems, and includes a unique combination of sensors, actuators, and AI algorithms that optimize the system's performance and energy usage. Present invention introduces a groundbreaking system, leveraging the latest advancements in artificial intelligence (Al) and deep learning. This initiative is designed to foster elderly independence, safety, and mental well-being within a supportive care environment. The application of the present invention sets a new standard in elderly care by integrating AI with insights from social sciences, ensuring a personalized approach that respects the autonomy of the elderly while providing families with peace of mind. The innovative use of AI for real-time fall detection and mental health assessments enables proactive safety interventions and addresses the emotional and psychological needs of the elderly, considering the complex interplay of social, psychological, and environmental factors. The solution provided stands out by offering a holistic care approach, rooted in a deep understanding of aging, societal integration, and the specific challenges faced by the elderly population in Switzerland and the rest of the world where elderly population is rapidly increasing. By monitoring, analyzing, and interpreting daily activities and potential safety risks, system of the present invention ensures a high-quality, fulfilling life for the elderly, aligning with the Sustainable Development Goals (SDGs).

The key innovations of the present invention are twofold: first, it advances computer vision-based fall detection and prediction technologies from TRL4-5 to a cost-effective TRL7-8 through novel research; second, it maximizes the benefits derived from visual information provided by camera-based systems while addressing the privacy issues that significantly hinder technology acceptance. The approach is innovative in several ways: (1) exploiting virtual reality to create representative and varied synthetic data for training new machine learning models; (2) using generative AI for the first time in this context to estimate occluded areas and enhance night vision capabilities; and (3) integrating the latest privacy-preservation methods in real-time.

The present invention provides a deep learning-based solution for fall detection and notification system, in order to quickly address the problem of an elderly person who lives alone, falling inside his/her living space. But it does not address only the critical issue of fall-related injuries, which account for 81% of severe injuries in the elderly, but also tackles mental health challenges, presenting a comprehensive solution to improve the well-being and quality of life for worlds' aging population. By merging cutting-edge technology with social science research, this solution represents a significant step forward in redefining elderly care standards, contributing to a safer, more inclusive, and healthier society. Thus additionally, the solution provided with the present invention aims to assess the mental health of elderly individuals and provide appropriate recommendations to enhance their overall well-being.

Therefore, the studies leading to the present invention have been dedicated to enhancing the safety and independence of world's elderly population, utilizing pioneering deep learning technology to develop an advanced fall detection and abnormality alert system. Amidst a demographic shift that sees a growing number of seniors living independently, often with chronic health issues and a heightened risk of accidents, the mission has been to provide a lifeline. By integrating a sophisticated network of sensors and a central hardware unit in homes, the system of the present invention monitors vital signs, facial expressions and movements continuously throughout the entire day and night, 24 hours a day, without interruption, ensuring immediate response in critical situations without compromising privacy. The solution lies in safeguarding the well-being of elderly individuals, ensuring they can live securely in their own homes, thereby fostering a future where independence doesn't compromise safety. The solution lies upon utilizing deep learning to identify habitual patterns of elderly individuals to correctly identify emergency cases (ie: accidental falls, life threatening circumstances such as accidents) targeting the elderly population.

Thus, the solution of the present invention is based on deep learning and data analysis techniques. Specifically, the focus of attention will be directed towards enhancing the well-being of senior citizens through the enhancement of emotion recognition and the identification of incidents involving falls among the elderly. The solution provided with the present invention is unique and innovative because, data from different sources is integrated and analyzed so that a recommendation based on a comprehensive analysis will be provided. An affordable and smart solution that will improve the quality of life of elderly people living alone at home is provided. Unlike traditional methods that may rely completely on wearable devices or manual alerts, system of the present invention utilizes strategically placed sensors and/or cameras in the living environment of the elderly. These cameras feed data into the deep learning algorithms, which are trained to recognize patterns of movement and identify when those patterns deviate in ways that signify a fall. This method ensures privacy and respect for the users' daily lives while providing comprehensive monitoring without requiring any action from the user.

Moreover, these intelligent living spaces have the ability to create digital twins of their residents while ensuring the utmost privacy and security. Additionally, their software features can be remotely expandable, thereby providing an agile solution that can adapt to the changing needs of their users.

These solutions would not only enhance the quality of life for the elderly but also ease the burden on healthcare systems and caregivers. Especially, technological progress has created opportunities for the development of high-performance solutions in this domain. Recent strides in sensor technology, machine learning, and wearable devices have notably enhanced the precision and user-friendliness of fall detection systems. The incorporation of artificial intelligence (Al) and the Internet of Things (IoT) has facilitated the creation of more sophisticated monitoring and alert systems. Moreover, advancements in natural language processing and facial recognition have bolstered the capacity to effectively identify and analyse emotions with greater accuracy.

The solution of the present inventions is meticulously tailored for the senior population, providing a comprehensive range of features, including daily activity recognition, smart home solutions, fall detection, and mental health assessment via emotion recognition. These elements are thoughtfully integrated to enhance the daily lives of seniors and oversee their well-being and health. In addressing the prevalent issue of falls, a primary concern for physical health, the objective is to respond to the critical requirement for enhanced safety and well-being among elderly individuals. The aim is to provide a comprehensive fall detection solution that promptly notifies caregivers or authorities in emergencies, effectively meeting this urgent need. The present invention also addresses the growing concern of mental health challenges among the elderly, which often go unnoticed. Through emotion recognition technology, the aim is to provide a means of assessing and addressing mental health issues effectively. By recognizing and responding to emotional states, tailored recommendations and interventions can be offered to improve emotional well-being and reduce feelings of isolation and loneliness. Additionally, by recognizing activities of daily living and smart home solutions, the intention is to provide a supportive environment that enables independent living, granting elderly individuals a sense of autonomy and security in their homes.

It also another aspect of the present invention to focus on refining the accuracy and responsiveness of the applications of this technology, ensuring they adapt seamlessly to individual needs. Additionally, increased user-friendliness is another aspect of the present invention, ensuring that elderly users find the system of the present invention intuitive and non-intrusive. The importance of privacy and data security is also recognized, and continuous efforts in these areas are essential. By actively seeking feedback from the users and remaining committed to ongoing research and development, the aim is to better meet the evolving needs of elderly individuals in aging populations while continuously striving for excellence in the solution of the present invention.

Customers can expect a multitude of benefits from the tailored solution of the present invention designed for the senior population all around the world. The comprehensive range of features, including daily activity recognition, smart home solutions, fall detection, and mental health assessment via emotion recognition, are meticulously integrated to significantly enhance the daily lives and overall well-being of elderly individuals. This solution is specifically designed to foster independent living, improving their overall quality of life in familiar home environments. Affordability and adaptability is a core aspect of the solution of the present invention, and it adds significant value to the offering. In addition to the comprehensive range of features and benefits, customers can expect the solution to be cost-effective and accessible. By providing an affordable alternative, the aim is to address the financial burden associated with elderly care in aging populations. The solution offers a sustainable and budget-friendly approach to caring for the elderly population, providing high-quality services without the exorbitant costs often associated with specialized care. This affordability ensures that a broader demographic of elderly individuals can access the support and monitoring they need to maintain their well-being and independence. By making the solution accessible to a wider audience, a more equitable and inclusive approach to elderly care is provided. Expensive care options will no longer be the sole recourse, as the solution of the present invention offers an effective and affordable means of improving the lives of elderly individuals and enhancing their overall quality of life.

Products and services, being the applications of the present invention will be provided through a subscription-based model. This approach aligns with the commitment to providing ongoing support and services to the elderly care solutions, ensuring that customers receive continuous benefits and updates. The subscription model offers several advantages, including predictable revenue streams, which will enable to sustain ongoing research and development, enhance the services, and ensure top-notch customer support. There is a willingness to pay from the targeted customers for several reasons. Firstly, the elderly population and their families prioritize the well-being and safety of their loved ones, making them more inclined to invest in solutions that enhance the quality of life and provide peace of mind. Secondly, the pricing strategy is designed to be competitive and affordable, making the solutions accessible to a wider range of customers. Thirdly, the value proposition of these services, including fall detection, mental health assessment, and smart home solutions, addresses critical needs in elderly care. Customers are likely to see the tangible benefits of these services, leading to a willingness to pay for ongoing support and peace of mind. Additionally, the plan is to offer different subscription tiers to cater to various customer preferences and budgets, further enhancing the appeal to a broader customer base. Overall, the subscription-based pricing model is well-suited to the target market and aligns with the value that is provided, making it a viable and attractive option for the customers. The essential features of the system according to the present invention are the following:
**Fall Detection:** Advanced deep learning algorithms are employed to detect falls, promptly alerting caregivers or emergency services.
**Emergency Contact Notification:** Should an emergency arise, system of the present invention ensures that ambulance services and family members are notified immediately.
**Family Monitoring:** A feature that allows family members to monitor the well-being of their elderly relatives, ensuring constant peace of mind.
**Medication Reminder:** An automated reminder system to aid the elderly in maintaining their medication schedule, which is crucial for managing health.
A user-friendly interface, ensuring ease of use for elderly customers is also an essential part of the invention. Additional features of the system include;
**Personal Reports:** Tailored health and activity reports are generated, providing insights into the user's well-being and any needed adjustments to their care plan.
**Emotional Recognition:** Employing advanced technology to recognize and respond to the emotional states of users, addressing the often-overlooked aspect of mental health in elderly care.
**Tracking:** stable tracking of individuals, an important aspect of monitoring in elderly care.
**Night Detection:** night detection capabilities, with a stable implementation.
**Data Collection:** With advanced CV/AI algorithms, data collection is also an important aspect, which is a crucial element for continuous improvement and personalized care.

The solution provided with the present invention for fall detection is based on local processing units/ anonymization techniques-video analysis/ floor sensors - pressure mats/ wearable sensors-accelerometers/ ML based solution- analyzing sound patterns or gait analysis (movement analysis). Since gait analysis for fall detection generates noisy data during the acquisition, in embodiments using gait analysis, a solution to the noise problem is also provided. The system of the present invention is designed in order to determine when an elderly individual experiences an event such as a fall which is detrimental to the monitored person. For this purpose, the system comprises a plurality of sensors worn by or proximate the monitored person. The proximate sensors of the present invention is adapted to detect a fall when the elderly individual is inside the living space however wearable sensors may also be necessary to detect an event when the person is outside the living space for a short walk or any similar activity. The sensors each are used for producing a sensed parameter value indicative of a physiological and / or physical condition of the monitored person, wherein at least one of the plurality of sensors comprises a motion sensing component for producing a motion parameter value representative of motion by the monitored person. The system also comprises an analysis component for analyzing the parameter values from the sensors to determine whether an event detrimental to the monitored person has occurred and an alerting component for issuing an alert upon occurrence of an event as determined by the analysis component, the alert received by the notification component; the notification component issuing a notification to one or more receiving components; and an application in the one or more of the receiving components for establishing communications link with the living space so that an operator of the receiving component can talk with the monitored person.

The preferred embodiment of the present invention leverages camera-based systems for fall detection. Using camera-based technology, a real-time solution is provided, by accurate detection of falls which is a major risk factor for the elderly, leading to significant health complications and even fatalities. This system ensures immediate alerts to caregivers and emergency services, facilitating swift action and potentially reducing the time to intervention in order to mitigate the impact of falls. Utilizing deep learning, the system can precisely identify unusual activity patterns that may indicate a fall. Deep learning plays a key role to differentiate between a fall and non-fall events that may be falsely categorized as a fall. By building a profile of daily behavior, anomalies can be more precisely identified. Moreover, the solution of the present invention addresses a fairly simple yet common problem in today's fall detection solutions - by not completely depending on a wearable device which may not be present when needed the most.

Social isolation is a growing concern among the elderly, exacerbating mental health issues such as depression and anxiety. This problem has been magnified by the COVID-19 pandemic, highlighting the need for effective solutions to support mental health and promote social interaction. Thus, beyond physical safety, the present invention introduces features aimed at enhancing mental well-being through emotional recognition. This not only helps in early detection of potential mental health issues but also encourages a more connected and supportive living environment for the elderly

The preferred solution provided with the present invention for emotion recognition is voice-based analysis. Amazon Alexa, Google Asisstant, IBM Watson are used as voice assistant; but diversity lacking in data, relying solely on voice analysis for emotion identification can result in inaccuracies due to the absence of contextual understanding. Additional sensors for emotional well - being are also used. The sensors and voice recognition technologies are configured to detect emotional distress or changes in mood. This novel approach addresses the critical but often overlooked mental health aspect, focusing on the challenges of social isolation and loneliness among the elderly.

Thus, visual based analysis is also used: Sky Biometry, Visage Technologies, and MorphCast are examples used in several embodiments of the present invention.

The solution provided with the present invention includes the analysis of facial expressions, voice intonation analysis, physiological analysis - heart rate, body temperature, ADL based (when a person is in stress, there is a specific pattern that he/she performs).

In an embodiment of the present invention, the combination of the following are used for the analysis: ADL (activities of daily living) + emotion recognition + fall detection + smart home solutions.

There are so many sensors in home for various tasks but separate trainings are required for each task. According to the present invention, how to combine weakly supervised machine learning techniques with activity recognition methods to obtain a system capable of recognizing user activity from sparse and heterogeneous sensor configurations is explored. The ultimate goal is to provide activity recognition capabilities without having to perform specific training for each smart home. It is planned to gather data and generate synthetic data.

According to the system of the present invention, it is planned to integrate data from several sources to reduce the impact of misinterpretation of the incoming image and to remove the obsolescence of data from a single source such as only a camera.

According to the system of the present invention, it is strived to provide a cost-effective intelligent solution capable of making suggestions or executing pre-set actions. This is achieved by synthesizing and analyzing data from multiple sources, including audio, visual (camera), and various sensors.

It has been found out that there is a lack of a dataset of elderly people for emotion recognition based on voice analysis. One key aspect is the development of a dataset specifically tailored to the elderly population, which is also currently lacking in the field of fall detection. By creating this dataset, the aim is to bridge a critical gap in the existing research, enabling more accurate and relevant model training for fall detection among the elderly. This dataset will reflect the unique characteristics and challenges associated with this demographic, contributing to the advancement of the field.

The prevailing challenge in the field of fall detection and emotion recognition is the lack of model generalizability. Typically, these models are highly data-specific and are meticulously tailored for the chosen dataset in a given study. Models are not generalizable. According to the present invention, a dataset is created that will reflect the elderly population.

The systems and methods described herein in the context of the present invention may provide and enable smart living spaces by control of home appliance and/or control of other devices. These systems and methods may utilize advanced data processing and/or artificial intelligence to provide smart space systems and methods that are capable of learning. Additionally, these systems and methods may integrate and interconnect devices within existing infrastructure and wired and wireless home automation networks. Some of the features described herein may utilize big data systems, machine learning and artificial intelligence algorithms, cloud computing technologies, and cloud services, for example.

The present invention has been devised to not only collect sensor data but also to process and analyze it, providing personalized recommendations to users. A notable attribute of the system is its high adaptability, which enables it to be customized to cater to the specific requirements of individual users. This unique feature facilitates the transformation of living spaces into digital twins that replicate users' preferences and behavior patterns. The present inventive technology is distinguished by its ability to consolidate all data sources pertaining to an individual's behavior. This aggregated data is subsequently transmitted to a data lake located either in the cloud or on-premises, depending on the privacy preferences of the user. Upon storage, machine learning algorithms deployed at the edge-node or in the cloud are employed to analyze behavior, identify recurring patterns, and compare against established best practices. This analysis culminates in the provision of personalized recommendations to the user, in order to guide their daily behavior. Moreover, the system's ability to remotely expand its software capabilities provides an agile solution that can swiftly adapt to changing user needs.

Systems and methods described herein may comprise one or more computers. A computer may be any programmable machine or machines capable of performing arithmetic and/or logical operations. In some embodiments, computers may comprise processors, memories, data storage devices, and/or other commonly known or novel components. These components may be connected physically or through network or wireless links. Computers may also comprise software which may direct the operations of the aforementioned components. Computers may be referred to with terms that are commonly used by those of ordinary skill in the relevant arts, such as servers, PCs, mobile devices, routers, switches, data centers, distributed computers, and other terms. Computers may facilitate communications between users and/or other computers, may provide databases, may perform analysis and/or transformation of data, and/or perform other functions. Those of ordinary skill in the art will appreciate that those terms used herein are interchangeable, and any computer capable of performing the described functions may be used. For example, though the term "server" may appear in the specification, the disclosed embodiments are not limited to servers.

In some embodiments, the computers used in the described systems and methods may be special purpose computers configured specifically for providing smart living spaces. For example, a server may be equipped with specialized processors, memory, communication components that are configured to work together to perform smart space control, integration, learning as described in greater detail below.

Computers may be linked to one another via a network or networks. A network may be any plurality of completely or partially interconnected computers wherein some or all of the computers are able to communicate with one another. It will be understood by those of ordinary skill that connections between computers may be wired in some cases (e.g., via Ethernet, coaxial, optical, or other wired connection) or may be wireless (e.g., via Wi-Fi, WiMax, 4G, or other wireless connection). Connections between computers may use any protocols, including connection-oriented protocols such as TCP or connectionless protocols such as UDP. Any connection through which at least two computers may exchange data may be the basis of a network.

The smart living system of the present invention is designed to enhance user experience and automate various aspects of daily living by leveraging the Internet of Things (IoT) and other advanced technologies. Data is obtained from a wide range of devices and resources, including surveillance cameras, smart doorbell, smart door lock, lidar sensor, smartwatches, temperature sensors, and smart light switches. All these devices are connected to the local area network (LAN) within the living space and transmit data to their respective databases.

Following data storage, data streaming technology is employed to retrieve data from the APIs of these devices. The extracted data is then stored in buckets and database, depending on the specific requirements for data retention and usage.

In addition to loT devices, several external APIs, such as AccuWeather, music streaming services, and video streaming platforms, provide valuable data to the system. Similar to the loT devices, data from these APIs is retrieved and stored for further processing.

Once the data is securely stored, various applications are executed on EMR clusters using PySpark. These applications include Smart Home Intruder Detection, Auto Door Lock, Thermostat Adjustment, Auto Light Switch, Vital Health KPI Tracking, and Mental Health Tracking. Each application utilizes specialized algorithms, as detailed in the algorithm section, to analyze the data and optimize the performance of the respective smart home features.

When necessary, users are notified of any critical updates or alerts through a mobile application or web interface. This comprehensive system design ensures seamless integration of advanced technology to provide users with an efficient, safe, and comfortable living experience in their smart living space, preferably being mobile. The systems that may be controlled in the smart living space of the present invention are grouped under security, energy efficiency, health and wellness, without being limited thereof.

The living space being an Al-enabled living space, of the present invention includes the following components:
Delicate Sensors: The system includes a variety of sensors that collect data on variables such as temperature, humidity, light, and air quality. These sensors are designed to be unobtrusive and to blend seamlessly into the living environment.

AI Algorithms: The system includes algorithms as explained in detail hereinbefore, that use machine learning and other techniques to process the sensor data and optimize the user's living environment. The algorithms are highly adaptable and can be customized to meet the unique needs of each user.

Other Components: The system includes other components such as actuators, user interfaces, and data storage and analysis systems. These components work together to create a seamless and highly optimized living environment for the user.

The Al-enabled living space system offers several advantages over existing solutions. For instance it is; Highly Adaptable: The system is highly adaptable and can be customized to meet the unique needs of each user. The algorithms use machine learning to continuously improve the system's performance over time.

Unobtrusive Sensors: The delicate sensors are designed to be unobtrusive and to blend seamlessly into the living environment. This allows for a more natural and comfortable living experience.

These are just a few examples of the high-tech features that are implemented in one or more embodiments of the systems of the present invention. Thus, the present invention should not be understood by being limited to these features. As technology continues to advance, it is expected to see even more innovative and sustainable solutions for design and construction. In relation to these another advantage set forth with the present invention relies in its ease of adaptability to emerging technologies. Especially when compared with the conventional settlements in apartment buildings in big cities together with big populations, the adaptability of the present invention to developments in technology appears as an important advantage.

An advantage of providing artificial intelligence components in the living space of the present invention is to allow the effective management and analysis of different types of collected data. Another advantage is better protection of the user's privacy.

In the absence of internet connection effective control over smart living system is maintained. When internet connection is lost, the smart living system of the present invention is managed by implementing several practical measures. First, the smart devices of the present invention are connected to a local network using protocols like Zigbee, Z-Wave, and Bluetooth, allowing them to communicate directly without the need for an external connection. Also, a home automation hub is incorporated, centralizing control and enabling offline functionality for seamless management. Moreover, use of manual control options are made available on many smart devices, such as physical switches, to operate them even in the absence of internet access. Through these proven methods, effective control over our smart home system is maintained despite the connectivity challenges.

A detailed description of the AI components and how they interact with the other components of the system is explained below:

### HEALTH AND WELLNESS

### Vital Health KPI Tracking

### Input Data

Heart rate: A smart watch can track the user's heart rate continuously or periodically, providing a time-series data of heart rate changes over time.

Activity data: The smart watch can track a user's physical activity, such as steps taken, calories burned, distance traveled, and active minutes.

Sleep data: The smart watch can track a user's sleep quality and quantity, including total sleep time, time in bed, and time spent in different stages of sleep.

ECG (Electrocardiography) data: The smart watches that can generate an electrocardiogram (ECG) to measure the electrical activity of the heart, providing data that can be used to detect irregular heart rhythms.

Blood oxygen level: The smart watches that can measure the user's blood oxygen level, which can be used to detect potential respiratory or cardiac problems.

Ambient noise level: The smart watch can measure the ambient noise level in the user's environment, which can be used to alert the user to potential hearing damage. Location data: The smart watch can track the user's location, which can be used to provide personalized recommendations based on local weather, events, or services. Occupancy data: This could be collected using occupancy sensors, motion sensors, or other similar technologies. It would communicate to the algorithm whether a room or space is currently occupied or not.

### Required IOT and Data Sources

Main health data are retrieved from smart watches which are listed above. Additionally motion data are retrieved from lidar sensor to track walking style and rhythm.

### AI Approach

### Training Process

The Critical KPI monitoring functionality employs two sophisticated algorithms: anomaly detection and rule-based alerts. The anomaly detection algorithm capitalizes on historical user data by applying the Autoencoders deep learning method to recognize unusual patterns. Autoencoders neural networks are designed to minimize errors in reconstructing input data, which effectively identifies anomalies. When an anomaly is discovered, the system promptly alerts the user through a mobile application, keeping them up-to-date on any irregular measurements.

### Algorithm Implementation

A two-fold approach is used in the solution of the present invention. Firstly, the user's walking patterns and tempo are tracked with a lidar sensor. A convolutional neural network (CNN) is employed, which is trained by each user of the living space of the present invention. This pre-trained CNN generates an array containing the user's unique walking traits for every lidar data point. This array is subsequently inputted into the Autoencoders network mentioned earlier to detect any walking anomalies that might signify an injury. If an anomaly such as an injury is detected, the user receives a notification through the mobile application.

### Integration

Lastly, a rule-based alert system is offered that does not rely on algorithms. Users supply with specific health information gathered from their smartwatches. When the submitted data falls outside the user-defined threshold in the mobile application, the system of the present invention sends an alert. For example, if a user sets a blood oxygen level threshold below 90%, system notifies the user when the reading drops below that value. This integration of user-defined rules, alongside the advanced algorithms of the present invention, enhance the overall user experience by providing personalized and timely notifications.

### Mental Health Tracking

### Input Data

User Streaming History: Collect data on the user's listening and viewing history, including information about the specific tracks, albums, artists, genres, and playlists for music, as well as movies, TV shows, and video genres for video streaming. Timestamps: Record the date and time of each streaming activity to understand patterns in the user's habits, such as preferred time of day or days of the week for streaming.

Playback Duration: Keep track of how long users spend listening to each track or watching each video to identify their level of engagement with the content.

Emotional Attributes: Analyze the emotional attributes of the content being streamed, such as sentiment (positive, negative, or neutral), tempo, and energy level for music, and the mood or theme of movies and TV shows.

User Preferences: Gather data on the user's preferred content, such as favorite artists, playlists, movie genres, and other preferences to establish a baseline for their typical streaming patterns.

User Interaction: Monitor user interactions with the streaming platform, such as likes, dislikes, shares, and comments, to gain insights into their emotional responses to the content.

User Demographics: Collect demographic information about the user, such as age, gender, and location, to help contextualize their streaming patterns and preferences. External Factors: Consider external factors that may influence a user's streaming habits, such as weather, time of year, or significant events.

### Required IOT and Data Sources

Music and video streaming technologies APIs like spotify, youtube, netflix etc.

### AI Approach

This is a multi-variable pattern recognition problem which can be solved with an Al-based mental health tracking solution for smart living systems. It can be designed using various data sources to analyze user emotions and offer personalized insights. By processing User Streaming History, Timestamps, Playback Duration, and Emotional Attributes, the AI system identifies patterns and correlations between the user's emotional state and streaming habits. Incorporating User Preferences and User Interaction data helps the system recognize deviations from the norm, potentially detecting emotional distress or mental health concerns. User Demographics and External Factors provide context for the user's streaming habits within a broader socio-cultural environment. Utilizing machine learning algorithms, the Al system continuously refines its understanding of the user's emotional well-being and generates personalized recommendations, such as calming music or uplifting movies, to support their mental health.

### Training process:

Data Labeling: To train a supervised machine learning model, labeled data is necessary. Self-reported mental health data from users are collected as well as collaborating with mental health experts to review and label the collected data. This labeled data will serve as the target variable for the algorithm of the present invention.

### Algorithm Implementation

In addressing the issue at hand, an approach is adopted that entails assessing mental health through the identification of patterns. Although traditional methodologies, such as linear regression and random forest, provide some assistance, they fail to accurately represent temporal pattern alterations. Consequently, stationary algorithms are not ideally suited for the model of the present invention or the problem under consideration. Instead, the Long Short-Term Memory (LSTM) algorithm is employed, which is adept at establishing connections between music and video content, as well as their antecedents, thereby enabling effective pattern detection.

### Integration

It is important to incorporate the APIs of various streaming platforms, including Spotify, YouTube, and Netflix, into the system of the present invention. As illustrated in the Internet of Things (IoT) architecture section, the data obtained from these APIs will be utilized to populate the database.

By integrating these streaming APIs, the wealth of information provided by each platform is leveraged, which will significantly enhance the ability to analyze patterns and trends in user behavior. This comprehensive dataset will serve as a critical component of the loT infrastructure, enabling the development of more robust and accurate predictive models.

To achieve this integration, it is necessary to first familiarize with each platform's API specifications and requirements. This involves understanding the unique authentication processes, data structures, and usage limitations for each service. Next, the application is registered with the respective developer portals to obtain the necessary API keys and access tokens.

Once the requisite credentials are obtained, proceed to develop wrapper functions or utilize existing libraries that facilitate seamless communication with each API. These functions will be responsible for extracting relevant data from the platforms, such as track details from music streaming services such as Spotify, video metadata from video sharing website such as YouTube, and show information from streaming services offering a wide variety of TV shows, movies and original content such as Netflix. After extracting the necessary data, preprocess it by cleaning, transforming, and standardizing it into a uniform format that can be easily ingested by the database of the present invention. Also needed to ensure that the loT architecture is capable of handling data from multiple sources and can accommodate various data types and structures.

Finally, it is crucial to be mindful of the rate limits and quotas imposed by each platform's API. Strategies are implemented, such as caching or staggered requests, to avoid exceeding these limits and ensure uninterrupted data access.

By following these steps, the streaming APIs are successfully integrated into the loT architecture of the present invention, thereby maximizing the potential of the data available for analysis and pattern recognition.

Figure 1 illustrates IOT (internet of things) architecture and the letters and the numbers in the drawing refer to:
Diagram Layers
A: Perception; B: Network; C: ML (machine learning) Applications; D: Infructure
1: loT Thing Camera; 2: loT Thing Door Lock; 3: Smart Phone; 4: The Smart Home Intruder Detection; 5: Auto Door Lock; 6: Thermostat Adjustment; 7: Auto Light Switch;
8: Vital Health KPI Tracking; 9: Mental Health Tracking; 10: Database (Amazon DynamoDB Accelarator); 11: Bucket; 12: EMR Cluster; 13: Amazon Kinesis Data Streams;
E: 3^{rd} Party Data Sources
14: Music Streaming; 15: Video Streaming; 16: Google IOT API; 17: Garmin IOT API
18: AccuWeather API; 19: Smart watch health data
F: User Interface

An example of an Al algorithm that could be used in a smart system of the present invention:
A reinforcement learning algorithm is used to optimize the energy usage of a smart system. The algorithm learns from the system's energy usage patterns and optimize its energy consumption by adjusting the system's settings and parameters. The reinforcement learning algorithm uses a reward function that incentivizes the system to minimize energy consumption while maintaining optimal performance.

The algorithm continuously receives input data from sensors in the system that monitor variables such as temperature, humidity, and occupancy levels. The algorithm then uses this data to make decisions on how to adjust the living space's systems, such as adjusting the thermostat or turning off lights when the living space is unoccupied. Over time, the algorithm learns which actions lead to the most energy savings and adjust its behavior accordingly.

Similarly, smart appliances that incorporate predictive AI algorithms can learn from the user's daily habits to optimize their performance and energy consumption. For example, a smart refrigerator can learn the user's eating habits and automatically adjust its temperature and settings to keep food fresher for longer periods. It can also suggest recipes based on the ingredients that are available inside the fridge, or remind the user when items are about to expire.

Another example of predictive AI technology about daily habits used in smart living spaces is a smart security system that uses AI to learn the daily routines of the occupants and detect any unusual behavior or activity. The system can use data from sensors, cameras, and other sources to identify patterns and anomalies in behavior and can alert the occupants or authorities if necessary. The system can also learn from false alarms and adjust its algorithms accordingly to reduce the likelihood of false positives in the future. Additionally, the system can provide insights into how occupants can improve their security, such as by adding additional lighting or reinforcing doors and windows in certain areas of the living spaces.

This AI algorithm could significantly reduce energy usage and costs, while maintaining optimal performance and user comfort.

According to an embodiment of the present invention, a smart living system for older adults and senior care, comprises,
a living space controller with a module integrated structure that combines multiple functions into a single, efficient unit comprising,
▪ more than one environmental motion sensor,
▪ more than one door and window sensor,
▪ more than one temperature and humidity sensor,
wherein the living space controller is adapted to:
- collect, analyze and process information on any movement within the living space,
- continuously monitor daily activity patterns,
- detect the opening and closing of doors and windows to ensure security,
- continuously monitor the indoor climate and ensure comfortable living conditions,
- monitor and control of sensor devices, drive control and electric switch device and to transmit information to the computer cloud data center through high-speed data collection, analysis and processing subsystems, data exchange and local area network technology,
a home intelligent management module for enhancing the smart home system comprising,
- more than one advanced motion sensor,
- more than one smart camera equipped with motion detection and video analytics configured to identify detrimental events and monitor the individual's well-being, with emergency contact notification features,
- more than one smart camera equipped with facial recognition configured for identifying the resident and monitoring expressions for emotional state assessment, with user consent,
- more than one pressure sensor installed in beds and chairs configured to monitor usage and detect prolonged inactivity;
- a centralized hub to connect and integrate all devices, centralize data collection and analysis, and provide an interface for remote monitoring by family members and/or caregivers;
wherein the home intelligent management module is connected to the computer cloud data center through a data link formed by a high-speed data acquisition, analysis and processing subsystem.

According to another embodiment, the smart living system further comprises; a plurality of precision sensors, actuators, smart devices, mobile applications, and AI algorithms configured to collect, process, analyze, and recommend sensor data;
computer vision-based fall detection and prediction technologies, advanced from TRL4-5 to a cost-effective TRL7-8 using machine learning models trained on synthetic data created via virtual reality;
generative AI for estimating occluded areas and improving night vision, enhancing the accuracy of fall detection and prediction in low-light conditions;
privacy-preservation integrated in real-time, including contextual design approaches, privacy by design, and privacy by context paradigms to ensure user privacy and trust; making the smart living system a highly adaptable and customizable system to meet the unique needs of each older adult user, suitable for integration into nursing homes, hospitals, senior homes, and conventional apartment flats.

According to a preferred embodiment, the computer vision-based fall detection and prediction technologies utilize:
- convolutional Neural Networks (CNN) for human pose estimation;
- transfer learning and synthetic data generated through virtual reality simulations to address training data insufficiencies;
- real-time image enhancement algorithms for better performance in low-light conditions.

According to a different embodiment the privacy-preservation include:
- privacy filters such as blurring, pixelating, and avatar replacements;
- contextual parameters to determine the type of visual protection based on the relationship with the observer, the time of day, the level of nudity, the activity being performed, and the room in which it occurs.

According to a further embodiment the system supports multimodal interfaces, comprising:
- voice, touch, and gesture controls for interaction with the system;
- interactive prototypes exploring different combinations of interface and interaction types for fall detection, prevention, and handling.

According to another embodiment, the system includes a dashboard interface for caregivers, providing:
- real-time monitoring and alerts for fall events and predictions;
- configuration options for privacy settings and sensor integration;
- annotations and documentation from co-design workshops with stakeholders to ensure user needs are addressed.

According to a different embodiment, the system is validated through:
- empirical qualitative inquiries, including field visits and interviews with stakeholders to identify current practices and privacy needs;
- lab and field evaluations involving end-users to measure the effectiveness, efficiency, and user experience of the system.

According to a preferred embodiment of the present invention, the home intelligent management module further comprises medication reminder functionality integrated into the system, including notifications through the centralized hub and mobile applications to allow family members and caregivers to monitor the system, receive alerts and notifications, and remotely control home devices.

According to another preferred embodiment, the home intelligent management module further comprises, smart locks to allow caregivers or emergency services to access the home in case of an emergency, ensuring the resident's safety and security. According to a different embodiment, the home intelligent management module further comprises smart smoke and carbon monoxide detectors to detect fire and CO levels, and send alerts to the resident and emergency contacts.

According to another embodiment, the home intelligent management module further comprises smart scales for tracking weight and BMI(Body Mass Index), and monitoring any change in health status.

According to a further preferred embodiment, the home intelligent management module further comprises voice assistants for hands-free communication and control of smart home devices.

According to a preferred embodiment, the home intelligent management module further comprises artificial intelligence and machine learning systems configured to ;
▪ analyze data from all sensors and devices of this system,
▪ detect anomalies,
▪ predict potential health issues, and
▪ improve monitoring accuracy over time.

According to another preferred embodiment, the home intelligent management module further comprises voice tone analysis to detect emotional changes.

According to a further embodiment, the system for providing a smart living space for elderly people, comprises:
an enclosed structural element comprising walls having an envelope structure comprising an inner layer, a structural layer, an insulation layer and an outer layer arranged sequentially from the inside to the outside of the enclosed structure,
a plurality of smart objects for the detection and processing of data, wherein each smart object is either for home security, energy efficiency, health or wellness and can be located within different areas inside or on the outer part of the enclosed structure relevant to its purpose, and operationally connected to each other having the same purpose of either security, efficiency, health or wellness; by means of a wireless network wherein each of the smart object comprises at least,
one sensor device for the collection of data from a pre-determined area of the living space in relation to the purpose of the smart object,
at least one storage unit of said collected data,
at least one processing unit with high computational capacity configured for the processing of said collected data,
at least one wireless communication unit configured for the communication with at least one of the other smart objects having the same purpose of either security, efficiency, health or wellness,
distribution and synchronization means of the data collected by smart objects within the same purpose group,
a local area network configured to receive data from the plurality of smart objects,
an artificial intelligence server in communication with the local area network comprising cluster services configured to:
   generate clusters of the data received from each of the smart objects; and perform processing comprising using a cluster to detect an anomaly in the smart object, identify the smart object, classify the smart object based on its purpose being either security, efficiency, health or wellness,
   determine a user behavior, determine a user mood, determine an energy consumption pattern, or create an automated action, or a combination thereof,
   generate an actuation command or a user notification according to an evaluation of applicable rules.

### Conclusion:

The Al-enabled living space system using AI algorithms provides a new and improved way of optimizing living spaces using artificial intelligence. The system includes delicate sensors, AI algorithms, and other components that work together to create a highly optimized and comfortable living environment for the user. The system is highly adaptable, unobtrusive, and energy-efficient, making it an ideal solution for a wide range of living environments.

An additional advantage is that all embodiments allow users to measure key metrics of their lives, allowing seamless improvement in every aspect.

## Claims

1. A smart living system for older adults and senior care, comprises,
a living space controller with a module integrated structure that combines multiple functions into a single, efficient unit comprising,
▪ more than one environmental motion sensor,
▪ more than one door and window sensor,
▪ more than one temperature and humidity sensor,
wherein the living space controller is adapted to:
- collect, analyze and process information on any movement within the living space,
- continuously monitor daily activity patterns,
- detect the opening and closing of doors and windows to ensure security,
- continuously monitor the indoor climate and ensure comfortable living conditions,
- monitor and control of sensor devices, drive control and electric switch device and to transmit information to the computer cloud data center through high-speed data collection, analysis and processing subsystems, data exchange and local area network technology,
a home intelligent management module for enhancing the smart home system comprising,
- more than one advanced motion sensor,
- more than one smart camera equipped with motion detection and video analytics configured to identify detrimental events and monitor the individual's well-being, with emergency contact notification features,
- more than one smart camera equipped with facial recognition configured for identifying the resident and monitoring expressions for emotional state assessment, with user consent,
- more than one pressure sensor installed in beds and chairs configured to monitor usage and detect prolonged inactivity;
- a centralized hub to connect and integrate all devices, centralize data collection and analysis, and provide an interface for remote monitoring by family members and/or caregivers;
wherein the home intelligent management module is connected to the computer cloud data center through a data link formed by a high-speed data acquisition, analysis and processing subsystem.

2. The smart living system of claim 1, further comprising;
a plurality of precision sensors, actuators, smart devices, mobile applications, and AI algorithms configured to collect, process, analyze, and recommend sensor data; computer vision-based fall detection and prediction technologies, advanced from TRL4-5 to a cost-effective TRL7-8 using machine learning models trained on synthetic data created via virtual reality;
generative AI for estimating occluded areas and improving night vision, enhancing the accuracy of fall detection and prediction in low-light conditions;
privacy-preservation integrated in real-time, including contextual design approaches, privacy by design, and privacy by context paradigms to ensure user privacy and trust; making the smart living system a highly adaptable and customizable system to meet the unique needs of each older adult user, suitable for integration into nursing homes, hospitals, senior homes, and conventional apartment flats.

3. The smart living system of claim 2, wherein the computer vision-based fall detection and prediction technologies utilize:
- convolutional Neural Networks (CNN) for human pose estimation;
- transfer learning and synthetic data generated through virtual reality simulations to address training data insufficiencies;
- real-time image enhancement algorithms for better performance in low-light conditions.

4. The smart living system of anyone of claims 1 to 3, wherein the privacy-preservation include:
- privacy filters such as blurring, pixelating, and avatar replacements;
- contextual parameters to determine the type of visual protection based on the relationship with the observer, the time of day, the level of nudity, the activity being performed, and the room in which it occurs.

5. The smart living system according to anyone of preceding claims, wherein the system is adapted to support multimodal interfaces, comprising:
- voice, touch, and gesture controls for interaction with the system;
- interactive prototypes exploring different combinations of interface and interaction types for fall detection, prevention, and handling.

6. The smart living system according to anyone of preceding claims, wherein the system includes a dashboard interface for caregivers, providing:
- real-time monitoring and alerts for fall events and predictions;
- configuration options for privacy settings and sensor integration;
- annotations and documentation from co-design workshops with stakeholders to ensure user needs are addressed.

7. The smart living system according to anyone of preceding claims, wherein the system is validated through:
- empirical qualitative inquiries, including field visits and interviews with stakeholders to identify current practices and privacy needs;
- lab and field evaluations involving end-users to measure the effectiveness, efficiency, and user experience of the system.

8. The smart living system according to anyone of preceding claims, wherein the home intelligent management module further comprises medication reminder functionality integrated into the system, including notifications through the centralized hub and mobile applications to allow family members and caregivers to monitor the system, receive alerts and notifications, and remotely control home devices.

9. The smart living system according to anyone of preceding claims, wherein the home intelligent management module further comprises, smart locks to allow caregivers or emergency services to access the home in case of an emergency, ensuring the resident's safety and security.

10. The smart living system according to anyone of preceding claims, wherein the home intelligent management module further comprises smart smoke and carbon monoxide detectors to detect fire and CO levels and send alerts to the resident and emergency contacts.

11. The smart living system according to anyone preceding claims, wherein the home intelligent management module further comprises smart scales for tracking weight and BMI (Body Mass Index) and monitoring any change in health status.

12. The smart living system according to anyone of preceding claims, wherein the home intelligent management module further comprises voice assistants for hands-free communication and control of smart home devices.

13. The smart home system according to anyone of preceding claims, wherein the home intelligent management module further comprises artificial intelligence and machine learning systems configured to;
▪ analyze data from all sensors and devices of this system,
▪ detect anomalies,
▪ predict potential health issues, and
▪ improve monitoring accuracy over time.

14. The smart home system according to anyone of preceding claims, wherein the home intelligent management module further comprises voice tone analysis to detect emotional changes.
